# EUROPEAN PATENT SPECIFICATION

(11) **EP 1 824 566 B1**
(45) Date of publication and mention of the grant of the patent: **17.02.2010**
(21) Application number: 05852367.1
(22) Date of filing: 28.11.2005
(51) Int. Cl.: A61Q 11/00, A61K 8/97, A61K 8/34

(54) **ORAL COMPOSITIONS CONTAINING EXTRACTS OF ROSMARINUS AND RELATED METHODS**
ORALE ZUSAMMENSETZUNGEN MIT EXTRAKTEN VON ROSMARIN UND ZUGEHÖRIGE VERFAHREN
COMPOSITIONS ORALES CONTENANT DES EXTRAITS DE ROMARIN ET METHODES ASSOCIEES

(30) Priority: 17.12.2004 US 637340 P; 24.10.2005 US 256861
(43) Date of publication of application: 29.08.2007
(73) Proprietor: Colgate-Palmolive Company, New York NY 10022-7499 (US)
(72) Inventor: TRIVEDI, Harsh, M., Somerset, New Jersey 08873 (US); XU, Tao, East Brunswick, New Jersey 08816 (US); WORRELL, Cortney, L., Plainfield, New Jersey 07060 (US); PANALIGAN, Kimberlee, Parlin, New Jersey 08859 (US)
(74) Representative: Jenkins, Peter David
(86) International application number: PCT/US2005/043063
(87) International publication number: WO 2006/065522

(56) References cited:
- EP-A- 0 321 180
- WO-A-01/12144
- WO-A-2004/073672
- US-A- 4 606 911
- US-A- 2004 067 204
- US-A1- 2004 241 133
- PATENT ABSTRACTS OF JAPAN vol. 007, no. 209 (C-186), 14 September 1983 (1983-09-14) & JP 58 109410 A (RAION KK), 29 June 1983 (1983-06-29)
- PATENT ABSTRACTS OF JAPAN vol. 016, no. 164 (C-0931), 21 April 1992 (1992-04-21) & JP 04 013630 A (OGAWA KORYO KK), 17 January 1992 (1992-01-17)
- PATENT ABSTRACTS OF JAPAN vol. 007, no. 248 (C-193), 4 November 1983 (1983-11-04) & JP 58 134013 A (RAION KK), 10 August 1983 (1983-08-10)
- DATABASE WPI Section Ch, Week 200470 Derwent Publications Ltd., London, GB; Class B04, AN 2003-144985 XP002380564 & KR 437 574 B (ADWIN KOREA CORP) 26 June 2004 (2004-06-26)
- DATABASE CAPLUS [Online] CHEMICAL ABSTRACTS SERVICE, COLUMBUS, OHIO, US; 1999, TSUNEDA FUMIHIKO: "Dentifrices containing oral deodorants and saliva secretion stimulants" Database accession no. 1999:420860
- DATABASE CAPLUS CHEMICAL ABSTRACTS SERVICE, COLUMBUS, OHIO, US; 1983, "Color stabilization by titanium dioxide in tooth pastes" Database accession no. 1983:563856

## Description

The present invention relates to toothpaste compositions, dentifrice compositions, mouthwash compositions, chewing compositions and edible oral strip compositions.

### BACKGROUND OF THE INVENTION

Dentifrice compositions are widely used in order to provide oral health. Dentifrices in the form of toothpaste, mouth rinses, chewing gums, edible strips, and the like have been formulated with a wide variety of active materials that provide a number of benefits to the user. Among these benefits are antibacterial, anti-inflammatory, and antioxidant properties. These properties of dentifrices make them useful therapeutic agents to prevent or treat a number of oral health conditions such as cavities, gingivitis, plaque, tartar, periodontal disease, and the like.

Rosemary extract includes ursolic acid and carnosic acid. In many cases, the extract is isolated from leaves of *Rosmarinus officinalis*. Another product from *Rosmarinus officinalis*, rosemary oil, is derived from steam extraction of flowering parts of the plant as well as the leaves. Although rosemary oil has been suggested for use in dentifrices along with other oils such as eucalyptol and menthol, it is to be noted that the chemical composition of rosemary oil differs a great deal from rosemary extract.
JP-A-58-109410, JP-A-4-013630 and JP-A-58-134013 disclose compositions for the oral cavity that contain rosemary extract.
KR-A-437574 discloses a cosmetic composition to improve acne.
US-A-2004/0241133 discloses a deodoriser for a mouth cavity.
EP-A-0321180 discloses oral compositions for suppressing mouth odors.

### BRIEF SUMMARY OF THE INVENTION

The present invention provides compositions according to claims 1, 6, 9, 11 and 13.
It has now been found that addition of 0.01 to 5 wt% rosemary extract to various dentifrice compositions results in tooth paste, mouth rinses, and other compositions that are suitable for treating and preventing a variety of oral disease including gingivitis, plaque build-up, and the like. Rosemary extract, containing major amounts or ursolic acid and carnosic acid, is added to dentifrice compositions so that the amount delivered to the oral cavity upon use is effective to provide an antibacterial, antioxidant, and/or anti-inflammatory effect. The components of rosemary extract are combined with an antibacterial effective amount of a halogenated diphenylether, such as triclosan, to provide enhanced activity.

For example, tooth paste typically contains about 0.1 to about 1 % or less by weight of rosemary extract along with conventional dentifrice components such as humectants, abrasives, anticaries agents, surfactants, flavorants, and the like. Chewing gums and edible strips contain comparable levels of rosemary extract while mouth rinses and mouthwashes are typically formulated containing lesser amounts.

It has been found that dentifrices formulated with the components of rosemary extract exhibit antibacterial, anti-inflammatory, and/or antioxidant properties, based both in vitro and in vivo.

Further areas of applicability of the present invention will become apparent from the detailed description provided hereinafter. It should be understood that the detailed description and specific examples, while indicating the preferred embodiment of the invention, are intended for purposes of illustration only and are not intended to limit the scope of the invention.

### DETAILED DESCRIPTION OF THE INVENTION

"Antibacterial activity" herein means activity as determined by any generally accepted in vitro or in vivo antibacterial assay or test.

"Anti-inflammatory activity" herein means activity as determined by any generally accepted in vitro or in vivo assay or test, for example an assay or test for inhibition of prostaglandin production or cyclooxygenase activity,

"Antioxidant activity" herein means activity as determined by any generally accepted in vitro or in vivo antioxidant assay or test.

An "oral surface" herein encompasses any soft or hard surface within the mouth including surfaces of the tongue, hard and soft palate, buccal mucosa, gums and dental surfaces. A "dental surface" herein is a surface of a natural tooth or a hard surface of artificial dentition including a crown, cap, filling, bridge, denture, dental implant and the like.

The term "inhibiting" herein with respect to a condition such as inflammation in an oral tissue encompasses prevention, suppression, reduction in extent or severity, or amelioration of the condition.

An oral care composition of the present invention can take any form suitable for application to an oral surface. In various illustrative embodiments the composition can be a liquid solution suitable for irrigating, rinsing or spraying; a dentifrice such as a powder, toothpaste or dental gel; a periodontal gel; a liquid suitable for painting a dental surface (e.g., a liquid whitener); a chewing gum; a dissolvable, partially dissolvable or non-dissolvable film or strip (e.g., a whitening strip); a wafer; a wipe or towelette; an implant; a dental floss; etc. The composition can contain active and/or carrier ingredients additional to those recited above.

In certain embodiments the composition is adapted for application to an oral surface of a small domestic animal, for example a cat or a dog. Such a composition is typically edible or chewable by the animal, and can take the form, for example, of a cat or dog food, treat or toy.

Classification herein of an ingredient as an active agent or a carrier ingredient is made for clarity and convenience, and no inference should be drawn that a particular ingredient necessarily functions in the composition in accordance with its classification herein. Furthermore, a particular ingredient can serve a plurality of functions, thus disclosure of an ingredient herein as exemplifying one functional class does not exclude the possibility that it can also exemplify another functional class.

In one embodiment, a tooth paste composition is provided that contains at least one humectant, at least one abrasive material, and an antibacterial effective amount of an antibacterial component comprising ursolic acid and carnosic acid. As the source of ursolic acid and carnosic acid, rosemary extract is used over a range of 0.01 % to 5% by weight, for example 0.01% to 2% by weight and 0.1% to 1% by weight in the toothpaste composition. The toothpaste composition further contains a halogenated diphenylether as another antibacterial agent, for example, triclosan.

The composition of the invention can be used in a method for inhibiting bacterial growth in the oral cavity of a subject animal. The method comprises applying to the oral cavity or oral surfaces of the subject animal a dentifrice composition comprising an antibacterial effective amount of an extract comprising carnosic acid and/or ursolic acid. In various embodiments, the dentifrice composition is in the form of toothpaste, mouth rinses, chewing gums, edible strips, and the like.

In another embodiment, the invention provides mouth rinses or mouth washes comprising water, flavorants, and at least one hydric component such as ethanol, glycerol, and sorbitol together with rosemary extract, prepared for example by ethanol extraction of the leaves of Rosmarinus officinalis , which is a source of carnosic acid and/or ursolic acid.

In another embodiment, the invention provides a chewing gum comprising a gum base and flavorants in addition to rosemary extract as described above. In yet a further embodiment, edible strips are provided that contain film forming polymers and optionally flavorants in addition to rosemary extract as described above.

In one aspect, the antibacterial component of the oral compositions of the invention comprises one or more organic compounds found in the leaves of *Rosmarinus officinalis.* Non-limiting examples of the organic compounds include ursolic acid, carnosic acid, rosmarinic acid, carnosol, and oleanolic acid. In one embodiment, the antibacterial components contain carnosic acid and optionally other ingredients. In various embodiments, the antibacterial component is a mixture of compounds obtained from alcohol extraction of the leaves of the rosemary plant. In commercial embodiments, extracts of the leaves of rosemary plants are sold as rosemary extract by, for example Sabinsa Corporation of Piscataway, New Jersey.

Rosemary extract contains carnosic acid, rosmarinio acid, ursolic acid, oleanolic acid, and other organic and inorganic materials. The plant material extracted from the leaves of *Rosmarinus officinalis* contains several constituents. Non-limiting examples of constituents include flavonoids such as diosmetin, diosmin, genkwanin, genkwanin-4'-methylether; 6-methoxy-genkwanin, luteolin, 6-methoxyluteolin, 6-methoxyluteolin, 6-mothoxy-luteolin-7-glycosida, 6- methoxyluteolin-7-methyl ether, hispidulin, apigenin etc.; phenolic acids, such as rosmarinic, labiatic, chlorogenic, neochlorogenic, and caffeic acids; carnosic acid; rosmaricine and isomaricine (reaction products of carnosic acid); triterpenic acids (mainly ursolic and oleanolic acids, with traces of 19α-hydroxyursolic, 2β-hydroxy-oleanolic and 3β-hydroxyureaa-12,20(30)-dien-17-oic acids); rosmanol; 7-ethoxyrosmanol; betulenic acid; and carnosol. In various commercial embodiments, rosemary extract is standardized to contain a minimum of 15% carnosic acid and 20% by weight ursolic acid.

The level of organic components in rosemary extract varies according to extraction procedure, extraction solvent, and other chemical variables, as well as the natural variability in the plant itself. Typically, the extract contains about 10% to about 40% by weight of ursolic acid and about 10% to about 25% by weight carnosic acid. In preferred embodiments, the extract contains 15% to 25% by weight carnosic acid and 20% to 40% by weight ursolic acid, preferably about 15% to 16% by weight carnosic acid and about 20% to 25% by weight ursolic acid. Thus, rosemary acid contains ursolic acid and carnosic acid as major components. In various embodiments, the extract also contains relatively lessor amounts of oleanolic acid, such as about 5% to 15% by weight.

Rosemary extract is prepared according to known methods by alcohol extraction of alcohol soluble components from the ground leaves of *Rosmarinus officinalis.*

Treatment levels of the antibacterial components in various oral compositions are chosen to deliver an effective amount to the oral surfaces of the subject animal in which the oral compositions are applied. For example, in toothpaste and tooth gels, suitable concentrations of the antibacterial component include 0.01% by weight to 5% by weight, for example 0.05-5% by weight, and particularly 0.1-0.3% by weight. At lower treat levels, the antibacterial and other effects of the composition tend to be less significant. On the other hand, at the higher end of the treat level, increasing the level tends not to increase the effectiveness by a concomitant amount.

For tooth powders, the treat levels are approximately the same as for toothpastes and gels, while for rinses and washes, the treatment levels tend to be less. For example, mouth rinses and mouth washes contain 0.01% to 2% by weight of the antibacterial component, for example from 0.01% to 0.6%, 0.01% to. 0.2%, and 0.01 to 0.05%. In addition, chewing gum, paint-on compositions, edible strips, and the like tend to be formulated with a wide range of concentration of rosemary extract or carnosic acid. In various embodiments, the level of rosemary extract or carnosic acid is similar to those in mouth rinses.

In one aspect, addition of rosemary extract at the treatment levels discussed above with respect to various oral compositions has the effect of adding the major component(s) of rosemary extract, such as carosic acid and ursolic acid, at treatment levels that are reduced from those given above by the percent by weight composition made up of the individual components. Thus, in one embodiment, the invention provides dentifrices comprising carnosic acid in oral compositions at treatment levels of 0.01% by weight to 5% by weight. In other embodiments, the invention provides oral compositions having as an antibacterial component, a combination of carnosic acid and ursolic acid, such that the total treat level of ursolic acid and carnosic acid components is as given above. In a preferred embodiment, the oral compositions of the invention contain rosemary extract in the amounts indicated.

In various embodiments, tooth paste and tooth gels are formulated containing at least one humectant, at least one abrasive material, and an antibacterial effective amount of an antibacterial component comprising ursolic acid and carnosic acid. In one embodiment, the compositions contain 0.01% to 5% by weight of rosemary extract, preferably 0.1% to 2% by weight rosemary extract. In various preferred embodiments, the tooth paste or tooth gel compositions contain 1% to 70% by weight of at least one humectant, and 1% to 70% by weight of at least one abrasive material, in addition to 0.1% to 2% by weight rosemary extract.

The tooth paste and tooth gel compositions further comprise an antibacterial agent selected from the group of halogenated diphenylether compounds. A non-limiting example of halogenated diphenylether compound is triclosan,

Toothpaste and tooth gel compositions are formulated with optional other ingredients, including without limitation anticaries agent, additional antibacterial agents, anticalculus or tartar control agents, anionic carboxylate polymers, viscosity modifiers, surfactants, flavorants, pigments, and the like.

In various embodiments, the compositions comprise an orally acceptable source of fluoride ions, which serves as an anticaries agent. One or more such sources can be present. Suitable sources of fluoride ions include fluoride, monofluorophosphate and fluorosilicate salts as well as amine fluorides, including olaflur (N'-octadecyltrimethylendiamine-N,N,N'- tris(2-ethanol)-dihydrofluorido).

As anticaries agent, one or more fluoride-releasing salts are optionally present in an amount providing a total of about 100 to about 20,000 ppm, about 200 to about 5,000 ppm, or about 500 to about 2,500 ppm, fluoride ions. Where sodium fluoride is the sole fluoride-releasing salt present, illustratively an amount of about 0.01% to about 5%, about 0.05% to about 1% or about 0.1% to about 0.5%, sodium fluoride by weight can be present in the composition.

In another embodiment the composition comprises an orally acceptable antimicrobial (*e*.*g*., antibacterial) agent other than the rosemary components described above. One or more such agents can be present.

Phenolic compounds useful herein illustratively include, subject to determination of oral acceptability, those identified as having anti-inflammatory activity by Dewhirst (1980), Prostaglandins 20(2), 209-222, but are not limited thereto. Examples of antibacterial phenolic compounds include 4-allylcatechol, *p*-hydroxybenzoic acid esters including benzylparaben, butylparaben, ethylparaben, methylparaben and propylparaben, 2-benzylphenol, butylated hydroxyanisole, butylated hydroxytoluene, capsaicin, carvacrol, oreosol, eugenol, guaiacol, halogenated bisphenolics including hexachlorophene and bromochlorophene, 4-hexylrosorcinol, 8-hydroxyquinoline and salts thereof, salicylic acid esters including menthyl salicylate, methyl salicylate and phenyl salicylate, phenol, pyrocatechol, salicylanilide, thymol, triclosan and triclosan monophosphate.

The at least one phenolic compound is optionally present in a total amount of about 0.01% to about 10% by weight. Illustratively the total concentration of the at least one phenolic compound in a toothpaste or gel dentifrice or mouth rinse of the present invention can be about 0.01% to about 5%, for example about 0.1% to about 2%, about 0.2% to about 1% or about 0.25% to about 0.5%.

Other suitable antibacterial agents include, without limitation, copper (II) compounds such as copper (II) chloride, fluoride, sulfate and hydroxide, zinc ion sources such as zinc acetate, zinc citrate, zinc gluconate, zinc glycinate, zinc oxide, zinc sulfate and , sodium zinc citrate, phthalic acid and salts thereof such as magnesium monopotassium phthalate, hexetidine, octenidine, sanguinarine, benzalkonium chloride, domiphen bromide, alkylpyridinium chlorides such as cetylpyridinium chloride (CPC) (including combinations of CPC with zinc and/or enzymes), tetradecylpyridinium chloride and N-tetradecyl-4-ethylpyridinium chloride, iodine, sulfonamides, bisbiguanides such as alexidine, chlorhexidine and chlorhexidine digluconate, piperidino derivatives such as delmopinol and octapinol, magnolia extract, grapeseed extract, menthol, geraniol, citral, eucalyptol, antibiotics such as augmentin, amoxicillin, tetracycline, doxycycline, minocycline, metronidazole, neomycin, kanamycin and clindamycin, and the like. A further illustrative list of useful antibacterial agents is provided in U.S. Patent No. 5,776,435 to Gaffar et al.
If present, these additional antimicrobial agents are present in an antimicrobial effective total amount, typically about 0.05% to about 10%, for example about 0.1% to about 3% by weight, of the composition.

In another embodiment the composition comprises an orally acceptable anticalculus agent. One or more such agents can be present. Suitable anticalculus agents include without limitation phosphates and polyphosphates (for example pyrophosphates), polyaminopropanesulfonic acid (AMPS), zinc citrate trihydrate, polypeptides such as polyaspartic and polyglutamic acids, polyolefin sulfonates, polyolefin phosphates, diphosphonates such as azacycloalkane-2,2-diphosphonates (*e.g.,* azacycloheptane-2,2-diphosphonic acid), N-methyl azacyclopentane-2,3-diphosphonio acid, ethane-1-hydroxy-1,1-diphosphonic acid (EHDP) and ethane-1-amino-1,1-diphosphonate, phosphonoalkane carboxylic acids and salts of any of these agents, for example their alkali metal and ammonium salts. Useful inorganic phosphate and polyphosphate salts illustratively include monobasic, dibasic and tribasic sodium phosphates, sodium tripolyphosphate, tetrapolyphosphate, mono-, di-, tri- and tetrasodium pyrophosphates, disodium dihydrogen pyrophosphate, sodium trimetaphosphate, sodium hexametaphosphate and the like, wherein sodium can optionally be replaced by potassium or ammonium. Other useful anticalculus agents include anionic polycarboxylate polymers. The anionic polycarboxylate polymers contain carboxyl groups on a carbon backbone and include polymers or copolymers of acrylic acid, methacrylic, and maleic anhydride. Non-limiting examples include polyvinyl methyl ether/maleic anhydride (PVME/MA) copolymers, such as those available under the Gantrez™ brand from ISP, Wayne, NJ. Still other useful anticalculus agents include sequestering agents including hydroxycarboxylic acids such as citric, fumaric, malic, glutaric and oxalic acids and salts thereof, and aminopolycarboxylic acids such as ethylenediaminetetraacetic acid (EDTA). One or more anticalculus agents are optionally present in the composition in an anticalculus effective total amount, typically about 0.01 % to about 50%, for example about 0.05% to about 25% or about 0.1% to about 15% by weight.

In various embodiments, the anticalculus system comprises a mixture of sodium tripolyphosphate (STPP) and a tetrasodium pyrophosphate (TSPP). In various embodiments, the ratio of TSPP to STPP ranges about 1:2 to about 1:4. In a preferred embodiment, the first anticalculus active ingredient, TSPP is present at about 1 to about 2.5% and the second anticalculus active ingredient, STPP is present at about 1 to about 10%.

In one embodiment, the anionic polycarboxylate polymer is present about 0.1% to about 5%. In another embodiment, the anionic polycarboxylate polymer is present about 0.5% to about 1.5%, most preferably at about 1% of the oral care composition. In one embodiment according to the present invention, the anticalculus system comprises a copolymer of maleic anhydride and methyl vinyl ether, such as for example, the Gantrez S-97 product discussed above.

In various embodiments, the ratio of TSPP to STPP to the synthetic anionic polycarboxylate ranges about 5:10:1 1 to about 5:20:10 (or 1:4:2). In one embodiment, the anticalculus system of the oral care composition comprises TSPP, STPP, and a polycarboxylate such as a copolymer of maleic anhydride and methyl vinyl ether at a ratio of about 1:7:1. In a non-limiting embodiment, the anticalculus system consists essentially of TSPP present at about 0.5% to about 2.5%, STPP present at about 1% to about 10%, and a copolymer of maleic anhydride and methyl vinyl ether present at about 0.5% to about 1.5%

In another embodiment the composition comprises an orally acceptable stannous ion source useful, for example, in helping reduce gingivitis, plaque, calculus, caries or sensitivity. One or more such sources can be present. Suitable stannous ion sources include without limitation stannous fluoride, other stannous halides such as stannous chloride dihydrate, stannous pyrophosphate, organic stannous carboxylate salts such as stannous formate, acetate, gluconate, lactate, tartrate, oxalate, malonate and citrate, stannous ethylene glyoxide and the like. One or more stannous ion sources are optionally and illustratively present in a total amount of about 0.01% to about 10%, for example about 0.1% to about 7% or about 1% to about 5% by weight of the composition.

In another embodiment the composition comprises an orally acceptable zinc ion source useful, for example, as an antimicrobial, anticalculus or breath-freshening agent. One or more such sources can be present. Suitable zinc ion sources include without limitation zinc acetate, zinc citrate, zinc gluconate, zinc glycinate, zinc oxide, zinc sulfate, sodium zinc citrate and the like. One or more zinc ion sources are optionally and illustratively present in a total amount of about 0.05% to about 3%, for example about 0.1% to about 1%, by weight of the composition.

In another embodiment the composition comprises an orally acceptable breath-freshening agent. One or more such agents can be present in a breath-freshening effective total amount. Suitable breath-freshening agents include without limitation zinc salts such as zinc gluconate, zinc citrate and zinc chlorite, α-ionone and the like.

In another embodiment the composition comprises an orally acceptable antiplaque, including plaque disrupting, agent. One or more such agents can be present in an antiplaque effective total amount. Suitable antiplaque agents include without limitation stannous, copper, magnesium and strontium salts, dimethicone copolyols such as cetyl dimethicone copolyol, papain, glucoamylase, glucose oxidase, urea, calcium lactate, calcium glycerophosphate, strontium polyacrylates and chelating agents such as citric and tartaric acids and alkali metal salts thereof.

In another embodiment the composition comprises an orally acceptable anti-inflammatory agent other than the rosemary components described above. One or more such agents can be present in an anti-inflammatory effective total amount. Suitable anti-inflammatory agents include without limitation steroidal agents such as flucinolone and hydrocortisone, and nonsteroidal agents (NSAIDs) such as ketorolac, flurbiprofen, ibuprofen, naproxen, indomethacin, diclofenac, etodolac, indomethacin, sulindac, tolmetin, ketoprofen, fenoprofen, piroxicam, nabumetone, aspirin, diflunisal, meclofenamate, mefenamic acid, oxyphenbutazone and phenylbutazone. One or more anti-inflammatory agents are optionally present in the composition in an anti-inflammatory effective amount.

Compositions of the inventions optionally contain other ingredients such as enzymes, vitamins and anti-adhesion agents. Enzymes such as proteases can be added for anti-stain and other effects. Non-limiting examples of vitamins include vitamin C, vitamin E, vitamin B5, and folic acid. In various embodiments, the vitamins have antioxidant properties. Anti-adhesion agents include solbrol, ficin, and quorum sensing inhibitors.

Among useful carriers for optional inclusion in a composition of the invention are diluents, abrasives, bicarbonate salts, pH modifying agents, surfactants, foam modulators, thickening agents, viscosity modifiers, humectants, sweeteners, flavorants and colorants. One carrier material, or more than one carrier material of the same or different classes, can optionally be present. Carriers should be selected for compatibility with each other and with other ingredients of the composition.

Water is a preferred diluent and in some compositions such as mouthwashes and whitening liquids is commonly accompanied by an alcohol, *e*.*g*., ethanol. The weight ratio of water to alcohol in a mouthwash composition is generally about 1:1 to about 20:1, for example about 3:1 to about 20:1 or about 4:1 to about 10:1. In a whitening liquid, the weight ratio of water to alcohol can be within or below the above ranges, for example about 1:10 to about 2:1.

In one embodiment a composition of the invention comprises at least one abrasive, useful for example as a polishing agent. Any orally acceptable abrasive can be used, but type, fineness (particle size) and amount of abrasive should be selected so that tooth enamel is not excessively abraded in normal use of the composition. Suitable abrasives include without limitation silica, for example in the form of silica gel, hydrated silica or precipitated silica, alumina, insoluble phosphates, calcium carbonate, resinous abrasives such as urea-formaldehyde condensation products and the like. Among insoluble phosphates useful as abrasives are orthophosphates, polymetaphosphates and pyrophosphates. Illustrative examples are dicalcium orthophosphate dihydrate, calcium pyrophosphate, β-calcium pyrophosphate, tricalcium phosphate, calcium polymetaphosphate and insoluble sodium polymetaphosphate. One or more abrasives are optionally present in an abrasive effective total amount, typically about 5% to about 70%, for example about 10% to about 50% or about 15% to about 30% by weight of the composition. Average particle size of an abrasive, if present, is generally about 0.1 to about 30 µm, for example about 1 to about 20 µm or about 5 to about 15 µm.

In a further embodiment a composition of the invention comprises at least one bicarbonate salt, useful for example to impart a "clean feel" to teeth and gums due to effervescence and release of carbon dioxide. Any orally acceptable bicarbonate can be used, including without limitation alkali metal bicarbonates such as sodium and potassium bicarbonates, ammonium bicarbonate and the like. One or more bicarbonate salts are optionally present in a total amount of 0.1 % to about 50%, for example about 1% to about 20% by weight of the composition.

In a still further embodiment a composition of the invention comprises at least one pH modifying agent. Such agents include acidifying agents to lower pH, basifying agents to raise pH and buffering agents to control pH within a desired range. For example, one or more compounds selected from acidifying, basifying and buffering agents can be included to provide a pH of about 2 to about 10, or in various illustrative embodiments about 2 to about 8, about 3 to about 9, about 4 to about 8, about 5 to about 7, about 6 to about 10, about 7 to about 9, *etc*. Any orally acceptable pH modifying agent can be used, including without limitation carboxylic, phosphoric and sulfonic acids, acid salts (*e*.*g*., monosodium citrate, disodium citrate, monosodium malate, etc.), alkali metal hydroxides such as sodium hydroxide, carbonates such as sodium carbonate, bicarbonates, sesquicarbonates, borates, silicates, phosphates (*e*.*g*., monosodium phosphate, trisodium phosphate, pyrophosphate salts, etc.), imidazole and the like. One or more pH modifying agents are optionally present in a total amount effective to maintain the composition in an orally acceptable pH range.

In a still further embodiment a composition of the invention comprises at least one surfactant, useful for example to compatibilize other components of the composition and thereby provide enhanced stability, to help in cleaning the dental surface through detergency, and to provide foam upon agitation, *e*.*g*., during brushing with a dentifrice composition of the invention. Any orally acceptable surfactant, most of which are anionic, nonionic or amphoteric, can be used. Suitable anionic surfactants include without limitation water-soluble salts of C₈₋₂₀ alkyl sulfates, sulfonated monoglycerides of C₈₋₂₀ fatty acids, sarcosinates, taurates and the like. Illustrative examples of these and other classes include sodium lauryl sulfate, sodium coconut monoglyceride sulfonate, sodium lauryl sarcosinate, sodium lauryl isoethionate, sodium laureth carboxylate and sodium dodecyl benzenesulfonate. Suitable nonionic surfactants include without limitation poloxamers, polyoxyethylene sorbitan esters, fatty alcohol ethoxylates, alkylphenol ethoxylates, tertiary amine oxides, tertiary phosphine oxides, dialkyl sulfoxides and the like. Suitable amphoteric surfactants include without limitation derivatives of C₈₋₂₀ aliphatic secondary and tertiary amines having an anionic group such as carboxylate, sulfate, sulfonate, phosphate or phosphonate. A suitable example is cocoamidopropyl betaine. One or more surfactants are optionally present in a total amount of about 0.01% to about 10%, for example about 0.05% to about 5% or about 0.1% to about 2% by weight of the composition.

In a still further embodiment a composition of the invention comprises at least one foam modulator, useful for example to increase amount, thickness or stability of foam generated by the composition upon agitation. Any orally acceptable foam modulator can be used, including without limitation polyethylene glycols (PEGs), also known as polyoxyethylenes. High molecular weight PEGs are suitable, including those having an average molecular weight of about 200,000 to about 7,000,000, for example about 500,000 to about 5,000,000 or about 1,000,000 to about 2,500,000. One or more PEGs are optionally present in a total amount of about 0.1% to about 10%, for example about 0.2% to about 5% or about 0.25% to about 2% by weight of the composition.

In a still further embodiment a composition of the invention comprises at least one thickening agent, useful for example to impart a desired consistency and/or mouth feel to the composition. Any orally acceptable thickening agent can be used, including without limitation carbomers, also known as carboxyvinyl polymers, carrageenans, also known as Irish moss and more particularly τ-carrageenan (iota-carrageenan), cellulosic polymers such as hydroxyethylcellulose, carboxymethylcellulose (CMC) and salts thereof, *e*.*g*., CMC sodium, natural gums such as karaya, xanthan, gum arabic and tragacanth, colloidal magnesium aluminum silicate, colloidal silica and the like. One or more thickening agents are optionally present in a total amount of about 0.01% to about 15%, for example about 0.1% to about 10% or about 0.2% to about 5% by weight of the composition.

In a still further embodiment a composition of the invention comprises at least one viscosity modifier, useful for example to inhibit settling or separation of ingredients or to promote redispersibility upon agitation of a liquid composition. Any orally acceptable viscosity modifier can be used, including without limitation mineral oil, petrolatum, clays and organomodified clays, silica and the like. One or more viscosity modifiers are optionally present in a total amount of about 0.01% to about 10%, for example about 0.1% to about 5% by weight of the composition.

In a still further embodiment a composition of the invention comprises at least one humectant, useful for example to prevent hardening of a tooth paste upon exposure to air. Any orally acceptable humectant can be used, including without limitation polyhydric alcohols such as glycerin, sorbitol, xylitol or low molecular weight PEGs. Most humectants also function as sweeteners. One or more humectants are optionally present in a total amount of about 1% to about 70%, for example about 1% to about 50%, about 2% to about 25%, or about 5% to about 15% by weight of the composition.

In a still further embodiment a composition of the invention comprises at least one sweetener, useful for example to enhance taste of the composition. Any orally acceptable natural or artificial sweetener can be used, including without limitation dextrose, sucrose, maltose, dextrin, dried invert sugar, mannose, xylose, ribose, fructose, levulose, galactose, corn syrup (including high fructose corn syrup and corn syrup solids), partially hydrolyzed starch, hydrogenated starch hydrolysate, sorbitol, mannitol, xylitol, maltitol, isomalt, aspartame, neotame, saccharin and salts thereof, dipeptide-based intense sweeteners, cyclamates and the like. One or more sweeteners are optionally present in a total amount depending strongly on the particular sweetener(s) selected, but typically about 0.005% to about 5% by weight of the composition.

In a still further embodiment a composition of the invention comprises at least one flavorant, useful for example to enhance taste of the composition. Any orally acceptable natural or synthetic flavorant can be used, including without limitation vanillin, sage, marjoram, parsley oil, spearmint oil, cinnamon oil, oil of wintergreen (methylsalicylate), peppermint oil, clove oil, bay oil, anise oil, eucalyptus oil, citrus oils, fruit oils and essences including those derived from lemon, orange, lime, grapefruit, apricot, banana, grape, apple, strawberry, cherry, pineapple, etc., bean- and nut-derived flavors such as coffee, cocoa, cola, peanut, almond, etc., adsorbed and encapsulated flavorants and the like. Also encompassed within flavorants herein are ingredients that provide fragrance and/or other sensory effect in the mouth, including cooling or warming effects. Such ingredients illustratively include menthol, menthyl acetate, menthyl lactate, camphor, eucalyptus oil, eucalyptol, anethole, eugenol, cassia, oxanone, α-inisone, propenyl guaiethol, thymol, linalool, benzaldehyde, cinnamaldehyde, N-ethyl-*p*-menthan-3-carboxamine, N,2,3-trimethyl-2-isopropylbutanamide, 3-(1-menthoxy)-propano-1,2-diol, cinnamaldehyde glycerol acetal (CGA), menthone glycerol acetal (MGA) and the like. One or more flavorants are optionally present in a total amount of about 0.01% to about 5%, for example about 0.1% to about 2.5% by weight of the composition.

In a still further embodiment a composition of the invention comprises at least one colorant. Colorants herein include pigments, dyes, lakes and agents imparting a particular luster or reflectivity such as pearling agents. A colorant can serve a number of functions, including for example to provide a white or light-colored coating on a dental surface, to act as an indicator of locations on a dental surface that have been effectively contacted by the composition, and/or to modify appearance, in particular color and/or opacity, of the composition to enhance attractiveness to the consumer. Any orally acceptable colorant can be used, including without limitation talc, mica, magnesium carbonate, calcium carbonate, magnesium silicate, magnesium aluminum silicate, silica, titanium dioxide, zinc oxide, red, yellow, brown and black iron oxides, ferric ammonium ferrocyanide, manganese violet, ultramarine, titaniated mica, bismuth oxychloride and the like. One or more colorants are optionally present in a total amount of about 0.001% to about 20%, for example about 0.01% to about 10% or about 0.1% to about 5% by weight of the composition.

In another embodiment, mouthwash or mouth rinse compositions are provided that contain water, one or more flavorants such as discussed above, one or more organic hydric compounds, and an antibacterial effective amount of an antibacterial composition as discussed above. In various embodiments, the mouthwash or mouth rinse compositions contain from 0.01 % to 5% by weight of an alcohol extract of the leaves of a plant containing ursolic acid and carnosic acid, such as *Rosmarinus, officinalis*. In preferred embodiments, the compositions contain 0.01% to 1% by weight of rosemary extract, for example 0.02% to 0.5% by weight The one or more organic hydric compounds are orally acceptable organic solvents such as, without limitation, ethanol and glycerol. Optionally, the mouthwash and mouth rinse compositions contain a surfactant to aid in dispersal of the flavorants and antibacterial compositions.

In various embodiments, the invention provides chewing gum compositions comprising a gum base and an antibacterial effective amount of an antibacterial effective composition such as discussed above. Chewing gum formulations typically contain, in addition, one or more plasticizing agents, at least one sweetening agent and at least one flavoring agent.

Gum base materials are well known in the art and include natural or synthetic gum bases or mixtures thereof. Representative natural gums or elastomers include chicle, natural rubber, jelutong, balata, guttapercha, lechi caspi, sorva, guttakay, crown gum, and perillo. Synthetic gums or elastomers include butadiene-styrene copolymers, polyisobutylene and isobutylene-isoprene copolymers. The gum base is incorporated in the chewing gum product at a concentration of about 10 to about 40% and preferably about 20 to about 35%.

In other embodiments, the oral compositions comprise an edible oral strip comprising one or more polymeric film forming agents and an antibacterial effective amount of an antibacterial composition as discussed above. The one or more polymeric film forming agents are selected from the group consisting of orally acceptable polymers such as pullulan, cellulose derivatives, and other soluble polymers including those well-known in the art.

In various embodiments, tooth paste or gel compositions of the invention contain rosemary extract, at least one humectant, a silica based abrasive compound and optionally anticalculus agents. The anti-calculus agents optionally comprise polyanionic carboxylate polymers as described above. Optionally the compositions contain anticaries agents such as sodium fluoride, stannous fluoride, mono fluorophosphates, and the like. In addition to the humectant, the compositions contain about 1% to about 40% by weight water.

In various embodiments, the compositions are effective against a combination of oral bacteria, as shown for example, in artificial mouth antiplaque study. In various embodiments, significant reductions in plaque development are seen in comparison to a negative control containing none of the antibacterial composition.

In various embodiments, the compositions also show antioxidant properties, for example as demonstrated in an LPO-CC assay carried out with formulated dentifrices, and/or also show clinical effectiveness *in vivo.* For example, in preferred embodiments, compositions of the invention show anti-gingival efficacy in a modified gingival margin plaque index determination. The protocol, known as MGMPI, has been published. Compositions including rosemary extract at an effective amount show significant improvements over a negative control. In other embodiments, compositions of the invention are also effective against plaque as shown in short-term clinical studies.

The invention is based in part on the discovery that when components such as found in extracts of *Rosmarinus officinalis* are added to dentifrice compositions containing antibacterial effective amounts of halogenated diphenylethers, the anti-inflammatory effect of the dentifrice composition is enhanced. Accordingly, the invention provides dentifrice compositions that contain both rosemary extract and halogenated diphenylethers such as, without limitation, triclosan, triclosan monophosphate and 2,2'-dihydroxy-5,5'-dibromodiphenylether.

Thus, compositions of the invention contain, in addition to the antibacterial components such as rosemary extract, an antimicrobial agent selected from the group of diphenylethers. In a preferred embodiment, the diphenylether compound is triclosan. It has been found that rosemary extract does not interfere with the antimicrobial activity of the halogenated diphenylether compounds. Specifically, in a preferred embodiment, rosemary extract does not interfere with triclosan availability in a formulated toothpaste.

Effectiveness of compositions containing both rosemary extract and diphenylether compounds is also shown in an artificial mouth study that shows an improved antiplaque effect as against a composition containing the diphenylether compounds alone.

Compositions containing rosemary extract and diphenylether compounds such as triclosan show, in preferred embodiments, an increased anti-inflammatory effect as compared to compositions containing no rosemary extract. In a preferred embodiment, there is a synergistic effect between the rosemary extract and the diphenylether compound such as triclosan, as demonstrated for example in a prostaglandin E2 enzyme immunoassay (PGE2).

In various embodiments, addition of rosemary extract to oral compositions containing diphenylether compounds has also been shown to increase the antioxidant properties of the oral compositions, as demonstrated for example in the LPO-CC antioxidant assay. Finally, incorporation of rosemary extract into oral compositions that contain diphenylether compounds, such as triclosan, has been shown to improve performance in clinical gingivitis and plaque studies.

### EXAMPLES

### Example 1 - Dentifrice formulation with no polyanionic carboxylate polymers.

**Table 1**

| **Component** | **% wt.** |
|---|---|
| H₂O | 38.4 - 38.6 |
| Humectant | 34.6 |
| Abrasive silica | 20 |
| Other additives | 6.7 |
| (fluoride source, | |
| sweetener, flavors, | |
| pigments, viscosity | |
| modifier, etc.) | |
| extract of Rosmarinus officinalis | 0.1-0.3 |

The composition shows antibacterial, anti-plaque, anti-gingivitis efficacy in *in vitro* and *in vivo* tests.

### Example 2 - Dentifrice containing a polyanion carboxylate polymer.

**Table 2**

| Table 2 | |
|---|---|
| | **% wt.** |
| **Component** | |
| H₂O | 35.4 - 34.6 |
| Humectant | 34.6 |
| Abrasive silica | 20 |
| SLS | 1.5 |
| Gantree^{®} polycarboxylate | 2 |
| Other additives | 6.2 |
| (fluoride source, | |
| sweetener, flavors, | |
| pigments, viscosity | |
| modifier, etc.) | |
| Rosemary extract | 0.1 - 0.3 |

The composition shows anti-plaque and anti-gingivitis activity.

### Example 3 - Mouth rinse composition

**Table 3**

| **Component** | **% wt.** |
|---|---|
| H₂O | 76 |
| ETOH | 22 |
| Surfactant, flavor | 1.95 |
| sweetener | |
| Rosemary extract | 0.05 |

### Example 4 - Mouth rinse composition

**Table 4**

| **Component** | **% wt.** |
|---|---|
| H₂O | 75 |
| ethanol | 22 |
| Gantrez^{®} polycarboxylate | 0.5 |
| Other additives | 2.4 |
| (surfactant, | |
| sweetener, flavors) | |
| Rosemary extract | 0.05 |
| Triclosan | 0.05 |

### Example 5 - Antiplaque efficacy

The compositions of Examples 1 and 2 show anti-plaque efficacy in an artificial mouth assay. For example, at 0.2% rosemary extract, the compositions show 53-54% reduction plaque relative to a negative control.

### Example 6 - Antioxidant effectiveness

The compositions of Examples 1 and 2 show antioxidant efficacy in an LPO-CC (lipid peroxide) assay. A negative control gives an optical density of about 0.81, while Example 1 gives about 0.72 and Example 2 gives about 0.74, when formulated with 0.3% by weight rosemary extract.

### Example 7a - Dentifrice composition with Triclosan

**Table 7**

| **Component** | **% wt.** |
|---|---|
| H₂O | 35.1-35.3 |
| Humectant | 34.6 |
| Abrasive silica | 20 |
| SLS | 1.5 |
| Triclosan | 0.3 |
| Gantrez^{®} polycarboxylate | 2 |
| Other additives | 6.2 |
| (sweetener, flavors, | |
| pigments, viscosity | |
| modifier, polyanionic | |
| carboxylate polymer) | |
| Rosemary extract | 0.1- 0.3 |

The above components are formulated into a dentifrice.
Example 7b - This composition is like Example 7a, but contains no triclosan.
Example 7c - This composition is like Example 7a, but contains no rosemary extract.
Example 8 - Compositions of Example 7a having 0.2% rosemary extract show improved performance in clinical gingivitis and plaque studies, compared to compositions like those of Example 9.
Example 9 - Compositions of Example 7a show improved anti-inflammatory effectiveness, as compared to compositions like those of Example 7b or 7c.

## Claims

1. A toothpaste composition comprising:
at least one humectant;
at least one abrasive material;
an anticaries agent comprising a fluorine releasing compound;
an antibacterial component comprising 0.01% to 5% by weight rosemary extract, the rosemary extract comprising ursolic acid and carnosic acid;
an antibacterial agent comprising a halogenated diphenylether; and
an anionic polycarboxylate polymer.

2. A composition according to claim 1, comprising 0.1% to 2% by weight of the rosemary extract.

3. A composition according to claim 2, comprising 1% to 70% by weight of the at least one humectant, and 1% to 70% by weight of the at least one abrasive material.

4. A composition according to any foregoing claim, wherein the halogenated diphenylether compound comprises triclosan.

5. A composition according to any foregoing claim, wherein the at least one humectant is selected from the group consisting of glycerol, propylene glycol, and sorbitol.

6. A toothpaste composition comprising:
a halogenated diphenylether;
0.01% to 5% by weight of rosemary extract;
1% to 70% by weight of a humectant; and
1% to 70% by weight of at least one abrasive material.

7. A composition according to claim 6, further comprising an anionic polycarboxylate polymer.

8. A composition according to claim 6 or claim 7, wherein the halogenated diphenylether comprises triclosan.

9. A dentifrice composition which is a mouth rinse or mouth wash composition comprising 0.01 % to 5% by weight of rosemary extract, a halogenated diphenylether, one or more flavorants, water, and one or more hydric compounds selected from the group consisting of ethanol, glycerol, sorbitol, and propylene glycol.

10. A composition according to claim 9, wherein the halogenated diphenylether comprises triclosan.

11. A dentifrice composition which is a chewing gum composition comprising a gum base, 0.01 to about 5% by weight of rosemary extract and a halogenated diphenylether.

12. A composition according to claim 11, wherein the halogenated diphenylether compound comprises triclosan.

13. A dentifrice composition which is in the form of an edible strip comprising one or more film forming polymers, 0.01 to about 5%, based on the total weight of the edible strip, of rosemary extract and a halogenated diphenylether.

14. A composition according to claim 13, wherein the halogenated diphenylether comprises triclosan.

## Patentansprüche

1. Zahnpastazusammensetzung, umfassend:
mindestens ein Feuchthaltemittel,
mindestens ein Scheuermittel,
ein Antikariesmittel, das eine Fluor-freisetzende Verbindung umfasst,
eine antibakterielle Komponente, die 0,01 Gew.-% bis 5 Gew.-% Rosmarinextrakt umfasst, wobei der Rosmarinextrakt Ursolsäure und Carnosinsäure umfasst,
ein antibakterielles Mittel, das einen halogenierten Diphenylether umfasst, und
ein anionisches Polycarboxylatpolymer.

2. Zusammensetzung nach Anspruch 1, umfassend 0,1 Ges.-% bis 2 Gew.-% des Rosmarinextrakts.

3. Zusammensetzung nach Anspruch 2, umfassend 1 Ges.-% bis 70 Gew.-% des mindestens einen Feuchthaltemittels und 1 Gew.-% bis 70 Gew.-% des mindestens einen Scheuermittels.

4. Zusammensetzung nach einem der vorgehenden Ansprüche, wobei die halogenierte Diphenyletherverbindung Triclosan umfasst.

5. Zusammensetzung nach einem der vorgehenden Ansprüche, wobei das mindestens eine Feuchthaltemittel aus der Gruppe bestehend aus Glycerin, Propylenglycol und Sorbit ausgewählt ist.

6. Zahnpastazusammensetzung, umfassend:
einen halogenierten Diphenylether,
0,01 Gew.-% bis 5 Gew.-% Rosmarinextrakt,
1 Gew.-% bis 70 Gew.-% eines Feuchthaltemittels, und
1 Gew.-% bis 70 Gew.-% mindestens eines Scheuermittels.

7. Zusammensetzung nach Anspruch 6, die weiterhin ein anionisches Polycarboxylatpolymer umfasst.

8. Zusammensetzung nach Anspruch 6 oder 7, wobei der halogenierte Diphenylether Triclosan umfasst.

9. Zahnreinigungszusammensetzung, die eine Mundspülung oder Mundwasch-Zusammensetzung ist und 0,01 Ges.-% bis 5 Ges.-% Rosmarinextrakt, einen halogenierten Diphenylether, einen oder mehrere Geschmacksstoffe, Wasser und eine oder mehrere Hydroxylgruppen-enthaltende Verbindungen enthält, die aus der Gruppe bestehend aus Ethanol, Glycerin, Sorbit und Propylenglycol ausgewählt sind.

10. Zusammensetzung nach Anspruch 9, wobei der halogenierte Diphenylether Triclosan umfasst.

11. Zahnreinigungszusammensetzung, die eine Kaugummizusammensetzung ist und eine Kaugummibasis, 0,01 bis etwa 5 Ges.-% Rosmarinextrakt und einen halogenierten Diphenylether umfasst.

12. Zusammensetzung nach Anspruch 11, wobei der halogenierte Diphenylether Triclosan umfasst.

13. Zahnreinigungszusammensetzung, die in Form eines eßbaren Streifens vorliegt, der ein oder mehrere Film-bildende Polymere, 0,01 bis etwa 5 Gew.-% auf Basis des gesamten Gewichts des eßbaren Streifens Rosmarinextrakt und einen halogenierten Diphenylether umfasst.

14. Zusammensetzung nach Anspruch 13, wobei der halogenierte Diphenylether Triclosan umfasst.

## Revendications

1. Composition de pâte dentifrice, comprenant :
au moins un humectant ;
au moins une matière abrasive ;
un agent anticarie comprenant un composé libérant du fluor ;
un composant antibactérien comprenant de 0,01 % à 5 % en poids d'extrait de romarin, l'extrait de romarin comprenant de l'acide ursolique et de l'acide carnosique ;
un agent antibactérien comprenant un éther de diphényle halogéné ; et
un polymère anionique de type polycarboxylate.

2. Composition selon la revendication 1, comprenant de 0,1 % à 2 % en poids de l'extrait de romarin.

3. Composition selon la revendication 2, comprenant de 1 % à 70 % en poids de l'au moins un humectant, et de 1 % à 70 % en poids de l'au moins une matière abrasive.

4. Composition selon l'une quelconque des revendications précédentes, dans laquelle le composé de type éther de diphényle halogéné comprend du triclosan.

5. Composition selon l'une quelconque des revendications précédentes, dans laquelle l'au moins un humectant est choisi parmi le groupe consistant en le glycérol, le propylèneglycol et le sorbitol.

6. Composition de pâte dentifrice, comprenant :
un éther de diphényle halogéné ;
de 0,01 % à 5 % en poids d'extrait de romarin ;
de 1 % à 70 % en poids d'un humectant ; et
de 1 % à 70 % en poids d'au moins une matière abrasive.

7. Composition selon la revendication 6, comprenant, en outre, un polymère anionique de type polycarboxylate.

8. Composition selon la revendication 6 ou la revendication 7, dans laquelle l'éther de diphényle halogéné comprend le triclosan.

9. Composition dentifrice qui est une composition pour le rinçage buccal ou le lavage buccal, comprenant de 0,01 % à 5 % en poids d'extrait de romarin, un éther de diphényle halogéné, un ou plusieurs agents aromatisants, de l'eau, et un ou plusieurs composés hydriques choisis parmi le groupe consistant en l'éthanol, le glycérol, le sorbitol et le propylèneglycol.

10. Composition selon la revendication 9, dans laquelle l'éther de diphényle halogéné comprend le triclosan.

11. Composition dentifrice qui est une composition de gomme à mâcher comprenant une gomme de base, de 0,01 à environ 5 % en poids d'extrait de romarin et un éther de diphényle halogéné.

12. Composition selon la revendication 11, dans lequel le composé de type éther de diphényle halogéné comprend le triclosan.

13. Composition dentifrice qui est sous la forme d'une bande comestible comprenant un ou plusieurs polymères filmogènes, de 0,01 à environ 5 %, par rapport au poids total de la bande comestible, d'extrait de romarin, et un éther de diphényle halogéné.

14. Composition selon la revendication de 13, dans laquelle l'éther de diphényle halogéné comprend le triclosan.
